Europäisches Patentamt

European Patent Office

Office européen des brevets

(11) Publication number: **0 265 228 B1**

# EUROPEAN PATENT SPECIFICATION

(45) Date of publication of patent specification: **20.05.92**

(51) Int. Cl.⁵: **A61K 7/40**, C08F 220/06, C08F 220/18

(21) Application number: **87309265.4**

(22) Date of filing: **20.10.87**

(54) Acrylic copolymer and skin protective and their use.

(30) Priority: **23.10.86 JP 253071/86**
**23.10.86 JP 253072/86**

(43) Date of publication of application:
**27.04.88 Bulletin 88/17**

(45) Publication of the grant of the patent:
**20.05.92 Bulletin 92/21**

(84) Designated Contracting States:
**AT BE CH DE ES FR GB GR IT LI LU NL SE**

(56) References cited:
**DE-A- 1 745 360**
**DE-A- 3 435 468**
**DE-B- 1 024 209**
**DE-C- 708 639**
**US-A- 3 590 118**

**CHEMICAL ABSTRACTS, vol. 85, no. 12, 20th September 1976, page 20, abstract no. 78629m, Columbus, Ohio, US; & JP-A-76 36 292 (TOA GOSEI CHEM ICAL INDUSTRY CO., LTD) 27-03-1976**

(73) Proprietor: **SHIONOGI SEIYAKU KABUSHIKI KAISHA trading under the name of**

SHIONOGI & CO. LTD.
12, 3-chome, Dosho-machi Higashi-ku
Osaka(JP)

Proprietor: **NISSHIN CHEMICAL CO., LTD.**
2-17-33 Kitago
Takefu-shi Fukui(JP)

(72) Inventor: **Saitoh, Izuma**
**1-2-33-601, Kamikoshien**
**Nishinomiya-shi Hyogo(JP)**
Inventor: **Kido, Shigeru**
**327-20, Mishima**
**Higashiosaka-shi Osaka(JP)**
Inventor: **Sasaki, Yoshio**
**Nisshin-Syataku-Kita 2, 10-17 Bandai-cho**
**Takefu-shi Fukui(JP)**
Inventor: **Shinohara, Syuichiro**
**Nisshin-Syataku-Kita 8, 10-17 Bandai-cho**
**Takefu-shi Fukui(JP)**

(74) Representative: **Hardisty, David Robert et al**
**BOULT, WADE & TENNANT 27 Furnival Street**
**London EC4A IPO(GB)**

**Description**

This invention relates to acrylic copolymers with very few impurities and compositions thereof. The compositions of acrylic copolymer are applicable onto the surface of the skin and form very thin but strong films. The skin protective compositions are less irritative to the skin because of the absence of surfactants. The thin film once formed is resistant to acidic or neutral environments but easily dissolved in a slightly alkaline environment. Thus, the film is easily washed away by lathering with a toilet soap and water but can be useful cosmetically or protectively.

Coating compositions to guard the skin from injury by chemicals or other irritants are known. They are designed to protect the skin by a thin film formed on the skin.

Traditionally, cellulose derivatives dissolved in a strong organic solvent such as acetone and ethyl acetate were used, but the organic solvents irritated the skin or the mucosa, and the formed coating films could not be removed easily from the skin.

Recently, a skin protective composed of n-butyl polyester/maleic acid and a plasticized ethyl cellulose as the main components (hereinafter, sometimes called the prior art protective) has been commercially available in the United States, but it is not allowed yet in practical use in Japan because it includes some problems in use such that its safety has not been proved.

This prior art protective forms a protective film between the skin and an extracorporeal cathether, adhesive tape, plaster, diaper or the like. Also usage to protect the hand from being soiled with grease is proposed.

The present inventors have strenuously searched for a polymer capable of covering the skin of housewives and dishwashers in restaurants, hospitals, and beauty salons who use neutral detergents. Acrylic copolymers have long been used in the medical field and are known to have high safety. But since all the existing acrylic copolymers are manufactured in routine polymerizations, such as solution polymerization and emulsion polymerization, such copolymers are rich in anionic surfactants, chain transfer agents and residual monomers. Accordingly, not only are they irritative to the skin and have problems with respect to environmental protection, but also their coating films become brittle and reduce their waterproof properties when surfactants exist, and they are hence not suitable for the purposes of this invention mainly aiming at impermeability of neutral detergents.

The study of methods for synthesizing copolymer emulsions without using water-solubilizing agents such as surfactants or the equivalent has been continued, but it has been reported that, in the use of this method in the polymerization of, for example, ethyl acrylate and acrylic acid when the quantity of acrylic acid exceeds 2 to 3 molar percent, the thus-formed copolymer emulsion becomes mechanically unstable tending to induce gelation (Matsumoto et al; Kobunshi Ronbunshu, vol. 32, No. 9, 1975).

Moreover, acrylic copolymers already known for pharmaceutical use (e.g., Eudragit made by Rohm Pharma, West Germany) have unsatisfactory aspects for the use that this invention aims at, as represented by their elongation and tensile strength.

The present invention provides a skin-protective acrylic copolymer of ethyl acrylate and methacrylic acid wherein the weight ratio of ethyl acrylate to methacrylic acid is in a range between 75:25 and 95:5, such copolymer containing therein residual monomer at 50 ppm or less and substantially no surfactant. It also provide a skin protective composition or agent consisting essentially of the following components:

a. an acrylic copolymer of ethyl acrylate and methacrylic acid wherein the weight ration of ethyl acrylate to methacrylic acid is in a range between 75:25 and 95:5, said copolymer containing therein residual monomer at 50ppm or less and substantially no surfactants 2 to 10%

b. cellulose derivative 0.2 to 2%; and

c. the necessary amount of an aqueous alcohol to make the whole to substantially 100%.

In order to achieve the purposes of this invention, it is ncessary to satisfy the following conditions in good balance. Such copolymers have not been developed in the prior art. The desired properties of copolymers for skin protectives, which is the aim of this invention, are as follows:

1) a thin coating film can be formed;

2) water resistance and solubility in alkali;

3) a stretchable pliable film can be formed, which adheres tightly to the skin;

4) contain few impurities such as residual monomers, and have neither odor nor iritation;

5) neutral detergents cannot be passed therethrough;

6) soluble in alcohol or water-containing alcohol; and

7) permeable to air and to water and yet the covered portion does not become stuffy or sticky either.

The present inventors, considering the above requirements found that a preferably copolymer suitable for this invention can be synthesized by using monomers comprising 75 to 95 parts by weight of ethyl acrylate (hereinafter occasionally abbreviated as EA) and 25 to 5 parts by weight of methacrylic acid (hereinafter sometimes abbreviated as MAA) in deionozed water, with persulfate as a polymerization initiator and polymerizing with heating, and if desired, using redox catalysts solely or in combinations with each other (e.g. hydrogen peroxide, ferrous sulfate and L-ascorbic acid), and post-treating (see later). The monomer unit of the copolymer of this invention is explained further in detail.

Ethyl acrylate is used in the copolymer in an amount so as to occupy from 75 to 95 parts by weight, preferably in a range of 80 to 90 parts by weight. EA appears to make the synthesized copolymer easily soluble in solvents such as alcohols and water-containing alcohols.

Methacrylic acid is used in the copolymer in an amount so as to occupy from 25 to 5 parts by weight, preferably in a range from 20 to 10 parts by weight. When the content of MAA is less than the said lower limit, the solubility in alkali decreases to an insufficient level, and in contrast when the content of MAA exceeds the said upper limit, there is a decrease of elongation or pliability. When the content of MAA exceeds 35 parts by weight, the copolymer becomes unstable, and in order to make it stable, an emulsifying agent or a suspending agent is needed.

Water resistance of the film obtained in accordance with this invention is superior to that of the prior art protective.

The reason why MAA is selected instead of acrylic acid is that, aside from the fact that MAA is said to have higher safety, it was found to be superior in water resistance and in the reduction of residual monomers. Another important factor in selecting MAA is that it was discovered that, when producing a copolymer with EA without using an emulsifying agent, the unsaturated carboxylic monomer can be used at a higher ratio when using MAA is a polymeric constituent than when using acrylic acid. As a result, a safer acrylic copolymer with fewer impurities can be obtained. Comparing with the prior art skin protective, the acrylic copolymer of this invention can form a protective film which is smoother to the touch without a strange feel.

The properties required for the copolymer for use as a skin protective can be regulated by changing the ratio of ethyl acrylate and methacrylic acid. For example, when desiring a film having a particularly high water-resistance, the content of MAA should be lower, and when intending to produce a film with a high solubility in alkali, the content of MAA should be higher.

A general method of manufacturing the acrylic copolymer of this invention is explained below. Deionozed water is heated in a closed reaction vessel which is purged with nitrogen. Persulfate which is preliminarily dissolved in deionozed water is added as a polymerization initiator, and monomers of EA and MAA are added over a desired period while stirring. Polymerization advances, and at the time when monomer addition ends, polymerization also comes almost to the end. In order to further decrease the residual monomer concentration, the reaction temperature should be maintained or raised higher while stirring (herein this process may be called "post-treatment"). By cooling the reaction solution, the desired copolymer is obtained as a dispersion.

As a persulfate, ammonium persulfate, sodium persulfate and potassium persulfate may be listed for example, and the persulfate can be added wholly at once, or portionwise or continuously. The amount of the persulfate should be determined at the most appropriate value depending, for example, on the polymer concentration, polymerization temperature, or polymerization duration, but generally it is approximately 0.2 to 4 parts by weight per 100 parts by weight of the monomer, preferably approximately 0.4 to 3 parts by weight. When it is less than the lower limit, failure in stability of the copolymer dispersion and an increase in residual monomers may be induced, and, in contrast, when it is over the upper limit, the water resistance of the coating film decreases.

In order to shorten the time required for the post-treatment, the temperature in the post-treatment should be raised, or persulfate should also be used in the post-treatment, or a small amount of a redox catalyst (such as a combined form of hydrogen peroxide, ferrous sulfate and L-ascorbic acid) should be added. If the aim is solely completion of the polymerization and the reduction of residual monomer, other polymerization initiators and redox catalysts than those stated above may be used, but considering the applications for the skin which are the aim of this invention, they are not preferred. Monomers, EA and MAA may be added separately but it is easier to add after mixing. Adding speed may vary, but adding at a constant speed is preferably because the formation of coarse particles can be prevented and the temperature can be regulated easily.

The polymerization temperature should be regulated in a range from about 45 to 98°C, preferably, from 65 to 95°C. When it is lower than the lower limit, coarse particles tend to be formed.

The copolymers concentration in the dispersion should not be specially limited but is preferably 15 to 65%, and more preferably, 25 to 55%. If the concentration is below the lower limit, the necessary amount of persulfate for monomer may increase or the relative amount of residual monomer may be enlarged. On the other hand, if above the upper limit, the amounts of coarse particles and deposits on the vessel wall increase because the copolymer dispersion becomes unstable.

According to the method explained above, copolymers in a variety of degrees of polymerization can be obtained. The weight-average molecular-weight is not critical, but for the purpose of this invention, it may be about 100,000 to 2,000,000 or preferably about 100,000 to 1,300,000. The molecular weight of formed copolymer can be freely selected by regulating the amount of persulfate, the polymerization temperature and copolymer concentration. Or if using an alcohol such as ethanol and isopropanol as a chain transfer agent, the molecular weight can be reduced. To adopt commonly used chain transfer agents, as represented by organic sulfur compounds, is not desirable in view of the safety of the skin.

The skin protectives of this invention can be prepared by dissolving the acrylic copolymers in an appropriate medium. An alcoholic solvent is preferable as the medium. Ethanol and water-containing ethanol are the most eligible dissolution media as a skin protective from the safety point of view, but if the content of ethanol is high, there are such problems as irritation of the skin having trauma because evaporation occurs quickly. On the contrary, if the water-content is high, the solubility of the copolymer is lowered or the evaporation speed drops. Isopropanol is usable in place of ethanol. A preferable mixing ratio of alcohol/water is from 60/40 to 80/20.

It is preferable to dissolve acrylic polymer in the medium at a concentration of about 2 to 10%, preferably about 4 to 7%. If it is above the upper limit, a stretched feeling may occur or the ability to adhere to the skin decreases while forming a coating. If it is below the lower limit, the formed coating film is thin and brittle, and is thus unfavorable. As for the cellulose derivative used in this invention, methyl cellulose, ethyl cellulose and hydroxypropyl cellulose may, for example, be listed. Among them, ethyl cellulose is preferred. The loadings of these celluloses are approximately 0.3 to 2%, preferably approximately 0.7 to 1.5%. At a ratio over the upper limit, the formed coating film is brittle and unfavorable. Moreover, below the lower limit, it is also unfavorable because of the adhesiveness while drying. The adhesiveness in coating and the stretchability after drying were obviously improved by the admixture.

For the skin protective of this invention, it is acceptable, if desired, to mix in appropriate softening agents, additives such as polyvinyl pyrrolidone, conservatives, coloring agents or pharmaceutically active ingredients. As the skin protective of this invention forms a colorless transparent thin coating film, it is very desirable from a cosmetic point of view. Depending upon the desired use, however, the film may be designed so as to permit checking for its presence by adding appropriate coloring agents.

The packaging forms which may be employed for the skin protective of this invention are not particularly limited. Since the protective is an alcoholic solution, a closed vessel capable of preventing the volatilization of alcohol is preferable. Bottles made of glass or plastic are acceptable as well as spray containers.

The benefits of the acrylic copolymer of this invention and the advantages in manufacturing the copolymer include, inter alia, the following:

(1) It forms a film which virtually prevents a neutral detergent from passing through, and which can be easily washed out by weak alkaline soap.

(2) It forms a thin film having a high stretchability and excellent pliability.

(3) Almost no odor and no irritation exist when the residual monomer level is 50 ppm or less. In ordinary emulsion polymerizations, much monomer is left over and thus the odor is strong. To reduce the residual monomer level a long time is normally needed for post-treatment because of the extension of the polymerization time. In the polymerization method of this invention, the post-treatment is easy as the residual monomer level is very low.

(4) As it is stable at ordinary temperature, it can be stored for a long period without using an emulsifying agent or a suspending agent.

The present invention is explained in more detail by the following Examples, which are not intended to limit the scope of the invention.

(Preparation of Copolymer)

Example 1

In a closed type reaction vessel with a stirrer which was purged with nitrogen, 236.1 parts by weight of deionized water was discharged, and after adjusting the temperature in the reaction vessel to 80°C, 1.2 parts by weight of ammonium persulfate was added and sequentially the following monomer mixture was added in 8 hours.

EA 85 parts by weight

MAA     15 parts by weight

Stirring was continued for another 8 hours by keeping the temperature in the reaction vessel at 80°C to complete the reaction. The solid content of the emulsion was 30%, in which EA was contained at 37 ppm. and MAA was less than 10 ppm according to the analysis of the residual monomer by means of gas chromatography. The mean molecular weight of this copolymer was about 840,000.

Example 2

In a closed type reaction vessel with a stirrer which was purged with nitrogen, 237.3 parts by weight of deionized water and 0.5 parts by weight of isopropanol were charged and after adjusting the temperature in the reaction vessel to 80°C, 1.7 parts by weight of ammonium persulfate was added and sequentially the following monomer mixture was added in 5 hours.

EA     85 parts by weight
MAA    15 parts by weight

By keeping the temperature in the reaction vessel at 85°C, 0.1 part by weight of hydrogen peroxide, ferrous sulfate, and L-ascorbic acid were added and stirring was continued for another 5 hours to complete the reaction. The solid content of this emulsion was 30%, in which EA was contained at 12 ppm, and MAA was less than 10 ppm according to the analysis of the residual monomer by means of gas chromatography. The mean molecular weight of this copolymer was about 180,000.

Example 3

In a closed type reaction vessel with a stirrer which was purged with nitrogen, 237.3 parts by weight of deionized water and 2.0 parts by weight of isopropanol were charged and after adjusting the temperature in the reaction vessel to 80°C, 1.7 parts by weight of ammonium persulfate was added and sequentially the following monomer mixture was added in 8 hours.

EA     80 parts by weight
MAA    20 parts by weight

By keeping the temperature in the reaction vessel at 85°C, 0.1 part by weight of hydrogen peroxide, ferrous sulfate, and L-ascorbic acid were added and stirring was continued for another 5 hours to complete the reaction. The solid content of this emulsion was 30%, in which EA was contained at 20 ppm, and MAA was less than 10 ppm according to the analysis of the residual monomer by means of gas chromatography. The mean molecular weight of this copolymer was about 130,000.

Example 4

In a closed type reaction vessel with a stirrer which was purged with nitrogen, 238.0 parts by weight of deionized water and 3.0 parts by weight of isopropanol were charged and after adjusting the temperature in the reaction vessel to 80°C, 2.0 parts by weight of ammonium persulfate was added and sequentially the following monomer mixture was added in 5 hours.

EA     75 parts by weight
MAA    25 parts by weight

By keeping the temperature in the reaction vessel at 85°C, 0.1 part by weight of hydrogen peroxide, ferrous sulfate, and L-ascorbic acid were added and stirring was continued for another 5 hours to complete the reaction. The solid content of this emulsion was 30%, in which EA was contained at 18 ppm, and MAA was less than 10 ppm according to the analysis of the residal monomer by means of gas chromatography. The mean molecular weight of this copolymer was about 110,000.

Example 5

In a closed type reaction vessel with a stirrer which was purged with nitrogen, 237.3 parts by weight of deionized water was charged and after adjusting the temperature in the reaction vessel to 80°C, 1.7 parts by weight of potassium persulfate was added and sequentially the following monomer mixture was added in 8 hours.

EA     85 parts by weight
MAA    15 parts by weight

By keeping the temperature in the reaction vessel at 80°C, 0.1 part by weight of hydrogen peroxide, ferrous sulfate, and L-ascorbic acid were added and stirring was continued for another 5 hours to complete the reaction. The solid content of this emulsion was 30%, in which EA was contained 15 ppm, and MAA was less than 10 ppm according to the analysis of the residual monomer by means of gas chromatography. The mean molecular weight of this copolymer was about 750,000.

Example 6

In a closed type reaction vessel with a stirrer which was purged with nitrogen, 187.8 parts by weight of deionized water was charged, and after adjusting the temperature in the reaction vessel to 75°C, 1.1 parts by weight of ammonium persulfate was added and sequentially the following monomer mixture was added in 5 hours.

EA      80 parts by weight
MAA     20 parts by weight

By keeping the temperature in the reaction vessel at 88°C, 0.1 part by weight of hydrogen peroxide, ferrous sulfate, and L-ascorbic acid were added and stirring was continued for another 5 hours to complete the reaction. The solid content of this emulsion was 35%, in which EA was contained at 15 ppm, and MAA was less than 10 ppm according to the analysis of the residual monomer by means of gas chromatography. The mean molecular weight of this copolymer was about 800,000.

Examples 7

In a closed type reaction vessel with a stirrer which was purged with nitrogen, 151.7 parts by weight of deionized water was charged, and after adjusting the temperature in the reaction vessel to 70°C, 1.1 parts by weight of ammonium persulfate was added and sequentially the following monomer mixture was added in 8 hours.

EA      85 parts by weight
MAA     15 parts by weight

By keeping the temperature in the reaction vessel at 80°C, 0.1 part by weight of hydrogen peroxide, ferrous sulfate, and L-ascorbic acid were added and stirring was continued for another 5 hours to complete the reaction. The solid content of this emulsion was 40%, in which EA was contained at 20 ppm, and MAA was less than 10 ppm according to the analysis of the residual monomer by means of gas chromatography. The mean molecular weight of this copolymer was about 1,180,000.

(Preparation of Skin Protective)

Example 8

The emulsion prepared in Example 1 was placed in an appropriate vessel so that the copolymer content in the emulsion is 50 g. Isopropanol (hereinafter occasionally abbreviated as IP) was added by 658 g and the mixture was stirred until the polymer was dissolved perfectly. To the solution of the polymer, ethylcellulose (hereinafter sometimes abbreviated as EC) was gradually added (10g) to prepare a solution, and water was added to make the whole 1kg. This composition contained the polymer of the Example 1 at 5.0% and EC at 1.0% in an aqueous IP solution.

Example 9

By using the copolymer prepared in Example 2, according to the preparation method of Example 8, an aqueous IP solution containing the copolymer 5.0% and EC 1.0% was prepared.

Example 10

By using the copolymer prepared in Example 3, according to the preparation method of Example 8, an aqueous IP solution containing the copolymer 5.0% and EC 1.0% was prepared.

Example 11

By using the copolymer prepared in Example 4, according to the preparation method of Example 8, an aqueous IP solution containing the copolymer 5.0% and EC 1.0% was prepared.

Example 12

By using the copolymer prepared in Example 1, according to the preparation method of Example 8, an aqueous IP solution containing the copolymer 3.5% and EC 1.0% was prepared.

Example 13

By using the copolymer prepared in Example 1, according to the preparation method of Example 8, an aqueous IP solution containing the copolymer 7.0% and EC 1.0% was prepared.

Example 14

By using the copolymer prepared in Example 1, according to the preparation method of Example 8, an aqueous IP solution containing the copolymer 10.0% and EC 1.0% was prepared.

Example 15

By using the copolymer prepared in Example 2, according to the preparation method of Example 8, an aqueous IP solution containing the copolymer 7.0% and EC 1.0% was prepared.

Experiment 1

The properties of the acrylic copolymer of this invention were examined by applying the following compositions on the back of the human hand. The results are shown in Table 1.

(Copolymer Tested)

Composition 1 (Invention):

To 26.4 parts by weight of emulsion prepared in Example 1, 65.4 parts by weight of isopropanol was added, and 9.2 parts by weight of purified water was added to prepare the solution for a skin protective.

Composition 2 (Invention):

To 26.4 parts by weight of emulsion prepared in Example 4, 65.4 parts by weight of isopropanol was added and 9.2 parts by weight of purified water was added to prepare the solution for a skin protective.

Composition 3 (Invention):

To 22.8 parts by weight of emulsion prepared in Example 6, 64.4 parts by weight of ethanol was added and 12.8 parts by weight of purified water was added to prepare the solution for a skin protective.

Reference Composition 1 (Prior Art):

Eudragit L30D-55 (acrylic copolymer made by Röhm Pharma, West Germany; concentration of solid content--30%), 26.4 parts by weight,-was dissolved in 64.4 parts by weight of isopropanol and 9.2 parts by weight of purified water was added to prepare the solution for a skin protective.

Table 1

| Item Tested / Sample Tested | Adhesiveness | Transparence | Spreadability | Contractility (Stiffness) | Water-Resistance | Solubility in Alkali | Permeability of Moisture | Overall Evaluation |
|---|---|---|---|---|---|---|---|---|
| Composition 1 | O | O | O | O | O | O | O | O |
| Composition 2 | O | O | O | O | O | O | O | O |
| Composition 3 | O | O | O | O | O | O | O | O |
| Ref. Comp. 1 | X | O | X | O | O | O | O | X |

(Note)　O : Good,　X : Not Good

Experiment 2

The properties of the composition of this invention were examined by applying the following compositions on the back of the human hand. As references, an EC-free composition of the polymer of this

8

invention, a composition with a known acrylic polymer and a commercial liquid adhesive plaster were used. The results are shown in Table 2.

(Composition Tested)

Test Compositions (The Present Invention):

   Compositions prepared in Examples 8 to 14

Compositions (including Prior Arts)

Composition 4

   The following component was contained in aqueous IP solution.
   Copolymer of Example 1     3.5%

Composition 5

   The following component was contained in aqueous IP solution.
   Copolymer of Example 1     5.0%

Composition 6

   The following component was contained in aqueous IP solution.
   Copolymer of Example 1     7.0%

Composition 7

   The following component was contained in aqueous IP solution.
   Copolymer of Example 1     10.0%

Prior Art Composition 1

   The following component was contained in aqueous IP solution.
   Eudragit E30D     5.0%
   EC                      1.0%

Prior Art Composition 2

   The following component was contained in aqueous IP solution.
   Eudragit E30D     10.0%
   EC                      1.0%

Prior Art Composition 3

   The following component was contained in aqueous IP solution.
   Eudragit L30D-55     10.0%
   EC                          1.0%

Prior Art Composition 4

   A commercially available film forming composition (aerosol type).

Prior Art Composition 5

   A commercially available liquid adhesive plaster.

Results

9

The skin protective of this invention showed obviously high organoleptic characteristics compared with a skin protecting using a known acrtlic polymer and a commercially available liquid adhesive plaster. Moreover, the adhesive property in coating and the spread-ability after drying were improved by the addition of cellulose.

Table 2

| Sample Tested | Item Tested | When Applied | | After Dried | | | | Overall Evaluation |
|---|---|---|---|---|---|---|---|---|
| | | Adhesiveness | Stickiness | Adhesiveness | Transparence | Stretchability | Stiffness | |
| INVENTION | Example 8 | O | O | O | O | O | O | O |
| | Example 9 | O | O | O | O | O | O | O |
| | Example 10 | O | O | O | O | O | O | O |
| | Example 11 | O | O | O | O | O | O | O |
| | Example 12 | O | O | O | O | O | O | O |
| | Example 13 | O | O | O | O | O | O | O |
| | Example 14 | O | O | O | O | O | O | O |
| EC-free | Comp. 4 | △ | △ | △ | O | O | O | △ |
| | Comp. 5 | O | △ | O | O | △ | O | O |
| | Comp. 6 | O | △ | O | O | △ | O | O |
| | Comp. 7 | △ | △ | O | O | △ | △ | △ |
| Prior Art | Prior Comp. 1 | O | △ | O | O | △ | O | O |
| | Prior Comp. 2 | O | × | O | O | △ | △ | △ |
| | Prior Comp. 3 | × | O | △ | O | × | O | × |
| | Prior Comp. 4 | O | △ | O | △ | × | × | × |
| | Prior Comp. 5 | O | O | O | △ | × | × | × |

(Note) O : Good, △ : Even, × : Not Good

10

Experiment 3

An organoleptic examination was performed on seven healthy adults by using the skin protective. The test method is stated below.

(Application of the Composition Tested)

According to the following criteria, the composition was applied on the right hand and the test composition on the left hand.

1. Five drops (approximately 100 mg) of a composition were put on the back of the right hand.

2. By using fingertips of the left hand, those drops were evenly spread from the center of the back of the right hand to the second joints of the four fingers.

3. Five drops (approximately 100 mg) of test composition were put on the back of the left hand.

4. By using fingertips of the right hand, those drops were evenly spread from the center of the back of the left hand to the second joints of the four fingers.

5. The state immediately after coating was evaluated.

6. The dry state of the composition was evaluated 10 minutes after dropping.

7. Sequentially, the state after using water was evaluated.

8. The compositions were removed by 70% ethanol.

9. Regarding the items 1 to 8 as one examination, three examinations a day were done for evaluation. Each interval between the neighboring courses should be longer than three hours.

(Items and Criteria for Evaluation)

[ Evaluation items ]

Immediately after Application

1. Easiness to spread
2. Stickiness
3. Easiness to dry
4. Total evaluation just after application

After drying

5. Gloss of the film formed
6. Smoothness of the film
7. Resistance to peeling-off
8. Twitch feeling to the skin
9. Total evaluation after drying

When using water

10. Resistance to peeling-off
11. Sliminess
12. Total evaluation when using water

Overall Evaluation

13. Overall evaluation throughout the above items.

[ Evaluation Criteria ]

Scores stated below were given on the respective items above, and analyzed according to the balance type incomplete block design.

Score

Poor: -2, Slightly Poor: -1, Even: 0, Slightly Good: +1, Good: +2.

According to the test method explained above, the organoleptic properties were compared on the compositions below. The results are shown in Table 3, where the scored values in the following tables indicate that, if it is larger than 0, the value is higher than the reference composition and or the contrary, if it is smaller than 0, the value is lower.

(Composition Tested)

Present Invention:

Skin-protectives prepared in Examples 8 and 9

Prior Art:

Prior Art Composition 6

Containing the following components in aqueous IP solution.
Eudragit E30D      3.5%
EC                        1.5%

Prior Art Composition 7

Containing the following components in aqueous IP solution.
Eudragit L30D-55    5.0%
Propylene glycol      1.0%

## Table 3

| Composition Tested / Test Items | Invention | | Prior Art | |
|---|---|---|---|---|
| | Example 8 | Example 9 | Prior Art 6 | Prior Art 7 |
| 1 | 0.095 | 0.381 | 0.095 | 0.238 |
| 2 | -0.143 | 0.714 | -0.143 | 0.286 |
| 3 | 0.048 | 0.333 | 0.047 | -0.381 |
| 5 | -0.095 | 0.048 | 0.048 | 0.048 |
| 7 | -0.048 | -0.048 | -0.333 | -0.048 |
| 8 | 0.095 | 0.952 | 0.952 | 0.381 |
| 13 | 0.095 | 0.476 | -0.095 | 0.048 |

Results

The skin protective of this invention showed high organoleptic characteristics compared with a skin protective using known acrylic copolymer.

Experiment 4

According to the method stated in Experiment 3, the preferred concentration of the copolymer in the skin protective was examined. The results are shown in Table 4.

(Composition Tested)

Example 9: copolymer 5.0%
Example 15: copolymer 7.0%

## Table 4

| Composition Tested | Example 9 | Example 15 |
|---|---|---|
| Test Items | | |
| 1 | 0.458 | 0.167 |
| 2 | 0.025 | -0.267 |
| 3 | 0.000 | 0.125 |
| 4 | 0.300 | -0.117 |
| 5 | 1.000 | 0.292 |
| 6 | 0.208 | -0.167 |
| 7 | 0.008 | -0.033 |
| 8 | 0.733 | -0.017 |
| 9 | 0.442 | -0.183 |
| 10 | -0.192 | -0.108 |
| 11 | -0.158 | -0.200 |
| 13 | 0.358 | -0.100 |

Results

The preferred concentration of the copolymer was 5%.

Effects of the invention

The skin protectives of this invention mainly made of acrylic copolymer were proved to be those which have a higher safety and better feel in use than any others in the prior art. The skin protective of the present invention forms a very thin coating film by applying only a very sill amount on the skin. The formed coating, film has a sufficient waterproof property, being stretchable and pliable, and adheres tightly to the skin without giving a strange feeling. Besides, because it contains substantially no surfactant and is designed to keep the amount of residual monomer to a minimum, it is less of an irritant and can prevent neutral detergents from passing through, and it is, hence, highly safe. And at the same time, after use, it can be easily removed by alcohol or weak alkaline solutions such as a soap, and so can present an excellent hand protective coating film for housewives, professional dishwashers, chemical workers, mechanics, and others.

Moreover, if an appropriate external skin agent is applied before using the skin protective of this invention, it can be useful as a liquid adhesive plaster or a protective coat in ODT treatment, especially helpful for the locations which normally contract and expand or are otherwise moved appreciably including hands, elbows, neck, face and the like.

## Claims

1.  An acrylic copolymer of ethyl acrylate and methacrylic acid wherein the weight ratio of ethyl acrylate to methacrylic acid is from 75:25 to 95:5, said copolymer containing residual monomer at 50 ppm or less and substantially no surfactant.

2.  An agent for protecting the skin comprising a copolymer as claimed in claim 1.

3.  An agent as claimed in claim 2 which consists essentially of the following components:
    a. acrylic copolymer as defined in claim 1 2 to 10%;
    b. cellulose derivative 0.2 to 2%; and
    c. an aqueous alcohol to make up the balance.

4.  An agent as claimed in claim 2 or claim 3, wherein the mean molecular weight of said acrylic copolymer is between about 100,000 and about 1,300,000.

5.  An agent as claimed in any one of claims 2 to 4, wherein said cellulose derivative is ethylcellulose.

6.  An agent as claimed in any one of claims 2 to 5, wherein said alcohol is ethanol or isopropanol.

7.  A process for preparing a skin protective copolymer as defined in claim 1 which comprises copolymerizing 75 to 95 parts by weight of ethyl acrylate and 25 to 5 parts by weight of methacrylic acid in the presence of an initiator followed by maintenance of at least the temperature employed during polymerization.

8.  A process as claimed in claim 7 wherein the copolymerization is carried out in deionized water and the initiator is a persulfate.

9.  The use of a copolymer as defined in claim 1 in making a skin protective agent as defined in any one of claims 2 to 6.

10. The skin cosmetic or protective use of an agent as defined in any one of claims 2 to 6.

## Revendications

1.  Copolymère acrylique d'acrylate d'éthyle et d'acide méthacrylique, dans lequel le rapport en poids acrylate d'éthyle/acide méthacrylique est de 75:25 à 95:5, ledit copolymère contenant 50 ppm ou moins de monomère résiduaire et substantiellement pas de tensio-actif.

2.  Agent de protection de la peau comprenant un copolymère selon la revendication 1.

3.  Agent selon la revendication 2, qui est constitué essentiellement des composants suivants :
    a. 2 à 10 % du copolymère acrylique tel qu'il est défini dans la revendication 1 ;
    b. 0,2 à 2 % d'un dérivé cellulosique ;
    c. une solution aqueuse d'alcool pour compléter à 100 %.

4.  Agent selon la revendication 2 ou 3, dans lequel le poids moléculaire moyen dudit copolymère acrylique est compris entre environ 100.000 et environ 1.300.000.

14

EP 0 265 228 B1

5. Agent selon l'une quelconque des revendications 2 à 4, dans lequel ledit dérivé cellulosique est l'éthylcellulose.

6. Agent selon l'une quelconque des revendications 2 à 5, dans lequel ledit alcool est l'éthanol ou l'isopropanol.

7. Procédé de préparation d'un copolymère pour la protection de la peau selon la revendication 1, comprenant la copolymérisation de 75 à 95 parties en poids d'acrylate d'éthyle et 25 à 5 parties en poids d'acide méthacrylique, en présence d'un initiateur, suivie du maintien au moins à la température utilisée pendant la polymérisation.

8. Procédé selon la revendication 7, dans lequel on effectue la copolymérisation dans de l'eau désionisée, et l'initiateur est un persulfate.

9. Utilisation d'un copolymère selon la revendication 1, pour préparer un agent de protection de la peau selon l'une quelconque des revendications 2 à 6.

10. Utilisation d un agent selon l'une quelconque des revendications 2 à 6 à des fins cosmétiques ou de protection de la peau.

**Patentansprüche**

1. Acrylcopolymer aus Ethylacrylat und Methacrylsäure, worin das Gewichtsverhältnis von Ethylacrylat zu Methacrylsäure 75:25 bis 95:5 beträgt, wobei das Copolymer Restmonomer mit 50 ppm oder weniger und im wesentlichen kein oberflächenaktives Mittel enthält.

2. Hautschutzmittel, enthaltend ein Copolymer gemäß Anspruch 1.

3. Mittel gemäß Anspruch 2, das im wesentlichen aus den folgenden Bestandteilen besteht:
   (a) 2 - 10% Acrylcopolymer gemäß Anspruch 1
   (b) 0,2 bis 2% Cellulosederivat und
   (c) Restmenge eines wässrigen Alkohols.

4. Mittel gemäß Anspruch 2 oder 3, worin das Durchschnittsmolekulargewicht des Acrylcopolymers ca. 100.000 bis ca. 1.300.000 beträgt.

5. Mittel gemäß jedem Anspruch 2 bis 4, worin das Cellulosederivat Ethylcellulose ist.

6. Mittel gemäß eines jeden der Ansprüche 2 bis 5, worin der Alkohol Ethanol oder Isopropanol ist.

7. Verfahren zur Herstellung eines hautschützenden Copolymers gemäß Anspruch 1, wobei man 75 bis 95 Gew.Teile Ethylacrylat und 25 bis 5 Gew.Teile Methacrylsäure in Gegenwart eines Initiators copolymerisiert, worauf man mindestens die während der Polymerisation angewandte Temperatur aufrechterhält.

8. Verfahren gemäß Anspruch 7, wobei man die Copolymerisation in deionisiertem Wasser durchführt und der Initiator ein Persulfat ist.

9. Verwendung eines Copolymers gemäß Anspruch 1 zur Herstellung eines Hautschutzmittels gemäß eines jeden der Ansprüche 2 bis 6.

10. Hautkosmetische oder -schützende Verwendung eines Mittels gemäß eines jeden der Ansprüche 2 bis 6.